# EUROPÄISCHE PATENTSCHRIFT

(11) **EP 4 051 945 B1**
(45) Veröffentlichungstag und Bekanntmachung des Hinweises auf die Patenterteilung: **30.09.2026**
(21) Anmeldenummer: 20800099.2
(22) Anmeldetag: 29.10.2020
(51) Int. Cl.: F16M 11/04, F16M 11/10, F16M 13/00, G06F 1/16, H04M 1/04, H04M 1/18, H04M 1/02

(54) **HANDHALTBARE VORRICHTUNG ZUR AUFNAHME VON LEBENDEN HAUTBEREICHEN MENSCHLICHER AUTOPODIEN UND VON DOKUMENTEN**
HANDHELD DEVICE FOR TAKING IMAGES OF LIVING SKIN REGIONS OF HUMAN AUTOPODIA AND DOCUMENTS
DISPOSITIF PORTATIF DE PRISE D'IMAGES DE RÉGIONS CUTANÉES VIVANTES DE DOCUMENTS ET D'AUTOPODES HUMAINS

(30) Priorität: 30.10.2019 DE 202019106022 U
(43) Veröffentlichungstag der Anmeldung: 07.09.2022
(73) Patentinhaber: Dermalog Jenetric GmbH, 07749 Jena (DE)
(72) Erfinder: WOLFER, Roberto, 07749 Jena (DE); FEMEL, Holger, 07751 Rothenstein (DE); VÖDISCH, Yvonne, 07749 Jena (DE); STANDAU, Jörg, 07743 Jena (DE)
(74) Vertreter: Gleim Petri Patent- und Rechtsanwaltspartnerschaft mbB
(86) Internationale Anmeldenummer: PCT/EP2020/080401
(87) Internationale Veröffentlichungsnummer: WO 2021/084011

(56) Entgegenhaltungen:
- US-A1- 2003 089 832
- US-A1- 2015 083 615
- MIKE CHAUSSEE: "A Portable Scanner Alternative for the iPhone - ScanJig!", 8 December 2016 (2016-12-08), pages 12 pp., XP055769156, Retrieved from the Internet <URL:https://ndassistive.org/blog/a-portable-scanner-alternative-for-the-iphone-scanjig/> [retrieved on 20210127]

## Beschreibung

Die Erfindung betrifft eine handhaltbare Vorrichtung zur Aufnahme von lebenden Hautbereichen menschlicher Autopodien und von Dokumenten. Sie findet insbesondere zur Personenidentifikation bei mobilen Einsätzen, z. B. bei einer Routine-Kontrolle von verdächtigen Personen, Zutritts- oder Anwesenheitskontrolle bei Veranstaltungen oder der Grenzkontrolle im Zug, Bus oder Flugzeug, statt. Aufgrund ihrer geringen Größe, ihrer Kompaktheit und des modularen Aufbaus kann sie von Kontrollbediensteten permanent mitgeführt werden und steht somit auch spontan für eine Personenidentifikation an einem beliebigen Ort zur Verfügung.

Die mobile Erfassung und Identifikation von Personen gewinnt stetig an Bedeutung. Hierfür werden Identitätsdokumente (ID-Dokumente) und / oder Fingerabdrücke von mehr als einem Fingerglied mit einem Bildaufnahmegerät aufgenommen und mit Datenbanken verglichen. Zur Aufnahme der Fingerabdrücke einer Person legt diese die Finger auf eine Auflagefläche des Bildaufnahmegerätes.

Ein Konzept für ein mobiles Fingerabdrucklesegerät ist in der gattungsgemäßen US 8 036 431 B1 beschrieben, das sich dazu eignet, von einem Kontrollbediensteten (z. B. Grenzbeamten) zu den zu identifizierenden Personen gebracht zu werden. Unabhängig davon, dass dieses Gerät nicht für die gleichzeitige Aufnahme mehrerer Finger ausgelegt ist und auch keine Dokumente über dieselbe Auflagefläche aufgenommen werden können, muss ein das Gerät bedienender Kontrollbediensteter bei wiederholender Verwendung stets eine gleiche Position zum Gerät einnehmen, um die Anzeige bequem betrachten zu können. Darüber hinaus bedarf es zum Transport zwischen den Einsätzen einer zumindest die Fingerauflagefläche schützenden Abdeckung.

Die US 2015/083615 A1 betrifft ein elektronisches Gerät, das mit einer magnetischen Befestigungsvorrichtung an einer Oberfläche befestigt werden kann. Die Vorrichtung kann ein Schutzgehäuse umfassen, das zur Aufnahme eines elektronischen Geräts geeignet ist. Das Schutzgehäuse kann so konfiguriert sein, dass es das elektronische Gerät vor Schäden durch Herunterfallen schützt. Das Schutzgehäuse kann einen weiblichen Befestigungsabschnitt enthalten, der in dem Schutzgehäuse angeordnet ist. Der weibliche Befestigungsabschnitt des Schutzgehäuses kann so konfiguriert sein, dass er magnetisch an einem männlichen Befestigungsabschnitt befestigt werden kann, der mit einer klappbaren Abdeckung, einer Oberflächenbefestigung oder einer Dockingstation verbunden ist. Nach der magnetischen Befestigung an der Klappabdeckung, der Oberflächenbefestigung oder dem Dock kann die Schutzhülle um den weiblichen Befestigungsabschnitt gedreht werden, damit das elektronische Gerät von einem Querformat in ein Hochformat und umgekehrt umgeschaltet werden kann.

Die US 2003/089832 A1 betrifft eine multifunktionale, gelenkige Vorrichtung mit einem einstellbaren Satz von drehbaren Verriegelungsblättern, die einen PDA oder ein anderes tragbares Gerät mit einer Handtragefähigkeit ausstatten kann, die einen sichereren Griff am PDA schafft, während die Hand noch für andere Aufgaben verwendet werden kann.

In Chaussee (Mike Chaussee: "A Portable Scanner Alternative for the iPhone - ScanJig!", 8. Dezember 2016 (2016-12-08), Seite 12 pp., XP055769156, Abrufbar unter: https://ndassistive.orf/blog/a-portable-scanner-alternative-for-the-iphonescanjig/) ist eine Vorrichtung zum Halten eines Smartphones gezeigt.

Es ist die Aufgabe der Erfindung, eine verbesserte handhaltbare Vorrichtung zur Aufnahme von lebenden Hautbereichen menschlicher Autopodien und von Dokumenten zu finden.

Diese Aufgabe wird erfindungsgemäß gelöst durch die Merkmale der unabhängigen Ansprüche. Vorteilhafte Ausgestaltungen sind in den Unteransprüchen angegeben.

Die Erfindung soll nachfolgend durch ein Ausführungsbeispiel näher beschrieben werden.

Die Zeichnungen zeigen:
- Fig. 1a: eine erste Ausführung einer erfindungsgemäßen Vorrichtung in einem aufgeklappten Zustand,
- Fig. 1b: die erste Ausführung der Vorrichtung in einem zugeklappten Zustand,
- Fig. 2: die einzelnen Module der ersten Ausführung der Vorrichtung,
- Fig. 3: eine Explosivdarstellung der Halteeinheit der ersten Ausführung der Vorrichtung, und
- Fig. 4: die erste Ausführung der Vorrichtung in dem aufgeklappten Zustand mit Abschlussstück.

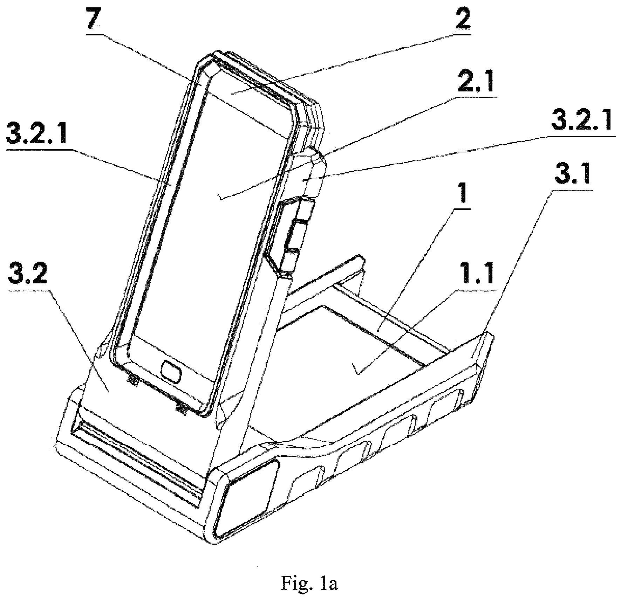

Alle Ausführungen einer erfindungsgemäßen Vorrichtung enthalten, wie in Fig. 1a dargestellt, ein Bildaufnahmemodul 1 und eine Halteeinheit 3 für ein Anzeige- und Bedienmodul 2.

Das Bildaufnahmemodul 1 enthält zumindest einen optischen, kapazitiven oder auf Ultraschall basierenden Scanner und eine Auflagefläche 1.1, auf der das zu scannende Objekt, insbesondere die Finger einer Person, aufgelegt werden, um ein Bild zu erzeugen.

Das Bildaufnahmemodul 1 kann des Weiteren eine Steuereinheit, eine Speichereinheit, eine Datenverarbeitungseinheit und eine interne Stromversorgungseinheit aufweisen.

Das Anzeige- und Bedienmodul 2 enthält zumindest ein Display 2.1, das optional als Touchscreen zur Bedienung bzw. Steuerung des Bildaufnahmemoduls 1 verwendet werden kann. Das Anzeige- und Bedienmodul 2 kann des Weiteren eine Steuereinheit, eine Speichereinheit, eine Datenverarbeitungseinheit und eine interne Stromversorgungseinheit aufweisen.

Die optional genannten Einheiten können in dem Bildaufnahmemodul 1, dem Anzeige- und Bedienmodul 2, teils in beiden Modulen oder extern, in keinem dieser Module, vorhanden sein. Das Bildaufnahmemodul 1 und das Anzeige- und Bedienmodul 2 können über hardwaremäßige Signal- oder Stromleitungen miteinander verbunden sein und/oder diese können miteinander und eventuell mit dritten elektronischen Modulen nur oder teilweise über drahtlose Verbindungen kommunizieren.

Die Halteeinheit 3 weist einen Basisrahmen 3.1 und einen Klapprahmen 3.2 auf, die über ein mechanisches Drehgelenk miteinander verbunden zueinander klappbar sind.

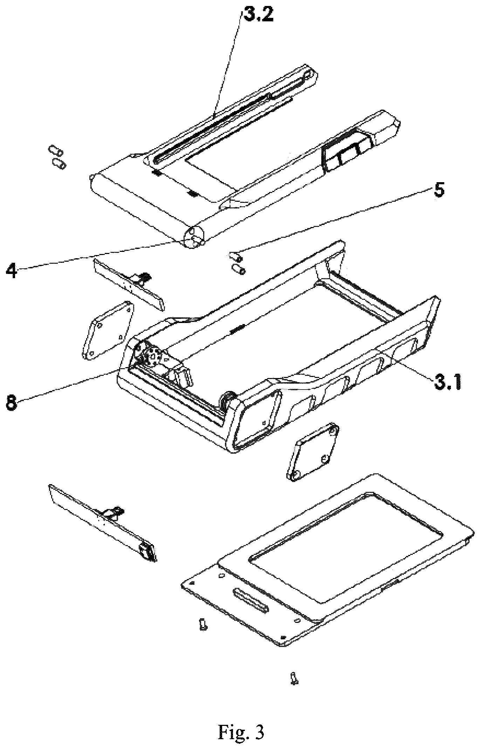

Das mechanische Drehgelenk kann durch eine Drehachse 4 bzw. zwei Zapfen 4 (s. Figur 3) gebildet sein, die mit dem Klapprahmen 3.2 fest verbunden sind und in dem Basisrahmen 3.1, optional in dort eingesetzten Rastscheiben 8, gelagert sind.

In dem Basisrahmen 3.1 ist das Bildaufnahmemodul 1 so angeordnet, dass dessen Auflagefläche 1.1 dem Klapprahmen 3.2 zugewandt ist. In dem Klapprahmen 3.2 ist das Anzeige- und Bedienmodul 2 so angeordnet, dass dessen Display 2.1 dem Basisrahmen 3.1 abgewandt ist.

Während des Betriebes, in dem ein Kontrollbediensteter durch Eingaben am Anzeige- und Bedienmodul 2 das Bildaufnahmemodul 1 steuert und das Display 2.1 betrachtet, ist die Vorrichtung auf einer Unterlage aufgestellt bzw. hält der Kontrollbedienstete die Vorrichtung in einem aufgeklappten Zustand (dargestellt in Fig. 1a) in einer Hand. Damit die Vorrichtung sicher in der Hand des Kontrollbediensteten liegt, können an dem Basisrahmen 3.1 Vertiefungen ausgebildet sein, in die er mit seinen Fingern greifen kann. Im aufgeklappten Zustand der Halteeinheit 3 schließen die Auflagefläche 1.1 und das Display 2.1 einen Winkel von kleiner 90° und größer 20° miteinander ein.

Zusätzlich oder alternativ zu den Vertiefungen kann an einer Unterseite des Basisrahmens 3.1 eine Schnittstelle für eine Haltevorrichtung, insbesondere für einen Kameragriff, vorgesehen sein, die es einem Nutzer der Vorrichtung ermöglicht, die Vorrichtung zu halten.

Mit dieser Winkeleinstellung innerhalb des angegebenen Bereiches ist ein sinnvoller Betrieb der Vorrichtung möglich. Die Winkel in diesem Bereich ermöglichen ein bequemes Betrachten durch den Kontrollbediensteten und führen dazu, dass die Person ihre Finger im Blick behalten kann, was in der Regel als angenehm empfunden wird.

Welcher Winkel konkret eingestellt wird, kann davon abhängen, in welchem Höhenabstand zur Augenhöhe des Kontrollbediensteten die Vorrichtung während des Betriebes steht bzw. durch ihn gehalten wird.

In dem aufgeklappten Zustand ist die Auflagefläche 1.1 für eine Person zum Auflegen seiner Finger frei zugänglich. Die Person kann, während ihre Finger auf der Auflagefläche 1.1 liegen, nicht ohne Weiteres auf das Display 2.1 schauen und kann somit nicht dort eventuell eingeblendete Kontrollergebnisse oder sonstige Informationen einsehen.

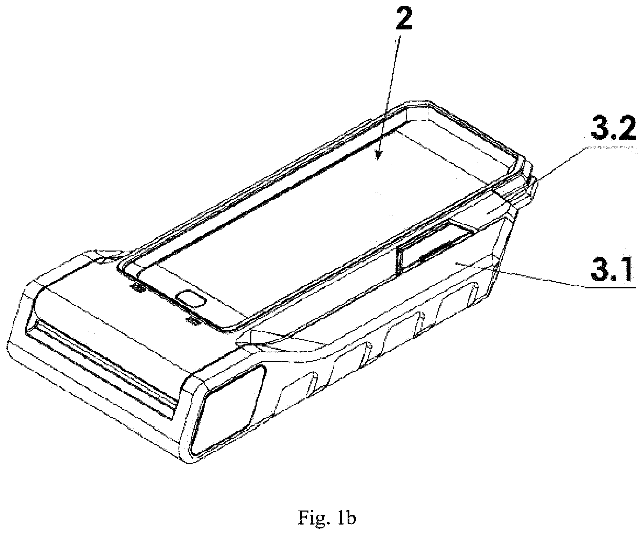

Außerhalb des Betriebes der Vorrichtung kann sich die Vorrichtung in einem zugeklappten Zustand der Halteeinheit 3 befinden, bei dem das Bildaufnahmemodul 1 von dem Anzeige- und Bedienmodul 2 abgedeckt und damit mechanisch geschützt ist, siehe Fig. 1b. Der zugeklappte Zustand der Halteeinheit 3 ist gegeben, wenn der Klapprahmen 3.2 auf dem Basisrahmen 3.1 aufliegt (nicht in den Zeichnungen dargestellt) oder, wie in Fig. 1b ersichtlich, innerhalb des Basisrahmens 3.1 liegt. Letzteres hat den Vorteil, dass sämtliche seitlich auf die Halteeinheit 3 einwirkende mechanische Kräfte, die beim Handling der Vorrichtung außerhalb des Betriebes auf sie einwirken können, von dem Basisrahmen 3.1 abgefangen werden, womit keine unerwünschten Kräfte oder Momente auf das Drehgelenk einwirken können.

Damit für das Betreiben in routinemäßiger Positionierung der Vorrichtung zum Kontrollbediensteten zwischen dem Display 2.1 und der Auflagefläche 1.1 wenigstens ein reproduzierbarer Winkel einstellbar ist, ist wenigstens ein Rastelement 5 mit einer Wirkungsrichtung parallel zur Drehachse 4 vorhanden, das entweder im Klapprahmen 3.2 gelagert und in den Basisrahmen 3.1 eingreifend oder im Basisrahmen 3.1 gelagert und in den Klapprahmen 3.2 eingreifend angeordnet ist. Gemäß einer ersten Ausführung der Vorrichtung weist diese, wie in Fig. 3 gezeigt, vier Rastelemente 5 auf, die paarweise jeweils einer in dem Basisrahmen 3.1 eingesetzten Rastscheibe 8 zugeordnet sind.

Die Rastscheiben 8 weisen eine identische Anordnung von Vertiefungen auf, in die die Rastelemente 5 eingreifen, sobald sie in eine hierzu fluchtende Position kommen. Die Rastelemente 5 können federnde Druckstücke mit einer Kugel sein. Während der Winkelverstellung zwischen dem Klapprahmen 3.2 und dem Basisrahmen 3.1 gleitet die Kugel bei gespannter Federung auf der Planfläche der Rastscheiben 8, bis sie in eine der Vertiefungen gerät und sich unter einer teilweisen Entspannung der Federung in diese einlegt. Die eingestellte Winkelposition entspricht einer Raststellung. Mit einem Druck auf den Klapprahmen 3.2 in Richtung des Basisrahmens 3.1 oder von diesem weg kann der Kontrollbedienstete eine weitere Raststellung einstellen oder auch die Halteeinheit 3 schließen. So wird auch erreicht, dass ein Schließen der Halteeinheit 3 ohne die Betätigung eines zusätzlichen Bedienelementes erfolgt.

Der notwendige Druck zur Änderung der Raststellung ist dabei mindestens so groß gewählt, dass während einer normalen Bedienung des Displays 2.1 durch den Kontrollbediensteten keine Veränderung der Raststellung eintritt, um eine ungewollte Veränderung der Raststellung während der Benutzung zu verhindern.

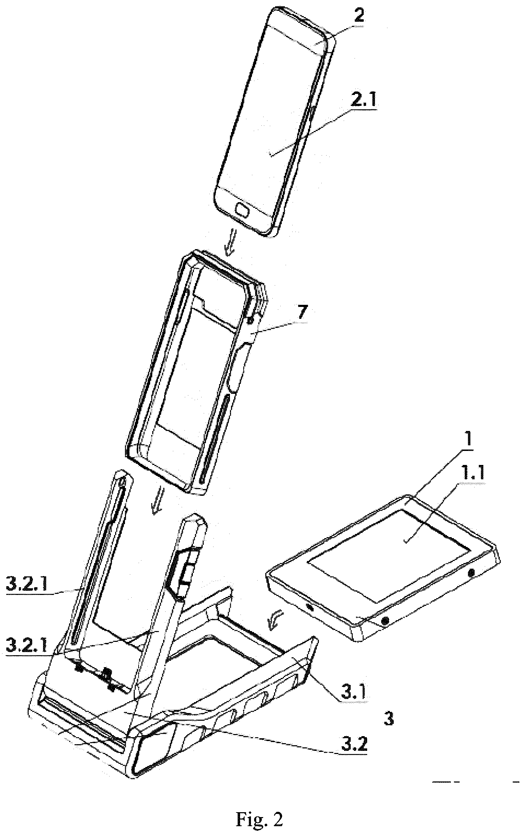

Um das Anzeige- und Bedienmodul 2 temporär, z. B. nur über die Dauer eines Einsatzes des Kontrollbediensteten, in die Halteeinheit 3 einzusetzen, kann der Klapprahmen 3.2 U-förmig mit zwei zueinander parallelen Schenkeln 3.2.1 ausgeführt sein, zwischen denen das Anzeige- und Bedienmodul 2 angeordnet ist, wie in Fig. 2 dargestellt. Das Anzeige- und Bedienmodul 2 kann so einfach von der offenen Seite des Klapprahmens 3.2 her in diesen in eine reproduzierbare Position geschoben werden.

Das Anzeige- und Bedienmodul 2 kann ein Smartphone sein.

Damit die Halteeinheit 3 für die Verwendung mit verschiedenen Smartphones kompatibel ist, können für Smartphones unterschiedlicher äußerer Abmessungen unterschiedliche Adapterhüllen 7 vorhanden sein, in denen jeweils das Smartphone eingesetzt, mittelbar im Klapprahmen 3.2 angeordnet ist.

Die Adapterhülle 7 kann aus einem biegsamen Material gefertigt, zum Beispiel Silikon oder thermoplastisches Polyurethan (TPU), und mit dem Klapprahmen 3.2 kraftschlüssig verbunden sein, indem sie, das Smartphone umschließend, zwischen die beiden Schenkel 3.2.1 gepresst ist.

Handelsübliche Smartphones weisen ihre Bedienelemente, z. B. einen Lautstärkeregler, einen Einschalttaster oder eine USB-Schnittstelle, an im Wesentlichen gleichen Stellen an ihrer Umfangsfläche auf, sodass eine jeweilige Zuordnung zu entsprechenden Bedienelementen an der Halteeinheit 3 über die Adapterhülle 7 oder zusätzliche Adapter möglich ist.

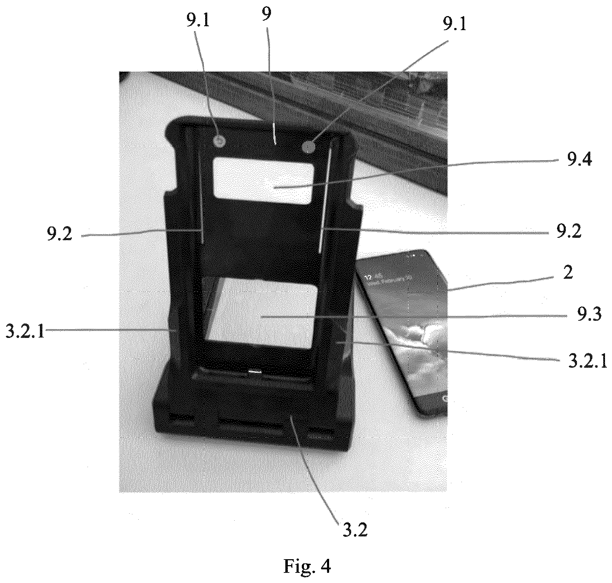

Zusätzlich oder alternativ zu dem Adapter 7 kann der Klapprahmen 3.2 an seiner Oberkante ein, insbesondere abnehmbar, z.B. mittels einer oder mehrerer Schraubverbindungen 9.1, zum Klapprahmen 3.1 verbundenes Abschlussstück 9 aufweisen, wie dies in Figur 4 dargestellt ist.

Das Abschlussstück 9 kann im Querschnitt im Wesentlichen L-förmig ausgeführt sein, sodass es mit den Schenkeln 3.2.1 des Klapprahmens 3.2 ein im Querschnitt im Wesentlichen O-förmiges Gehäuse für das Anzeige- und Bedienmodul 2 bildet.

Zum Einsetzen und Entnehmen des Anzeige- und Bedienmoduls 2 in bzw. aus dem Klapprahmen 3.2 kann das Abschlussstück 9 vom Klapprahmen 3.2 abgenommen werden.

Denkbar ist auch, dass das Abschlussstück 9 zusätzlich oder alternativ so biegsam ausgeführt ist, dass es zum Einsetzen und Entnehmen des Anzeige- und Bedienmoduls 2 in bzw. aus dem Klapprahmen 3.2 aus einer Einsetz- bzw. Entnahmerichtung des Anzeige- und Bedienmoduls 2 gebogen werden kann. Dazu kann das Abschlussstück 9 ein elastisches Material aufweisen. Zusätzlich oder alternativ kann ein Scharnier vorgesehen sein, um das Abschlussstück 9 zum Einsetzen und Entnehmen des Anzeige- und Bedienmoduls 2 in bzw. aus dem Klapprahmen 3.2 aus der Einsetz- bzw. Entnahmerichtung des Anzeige- und Bedienmoduls 2 zu schwenken.

Zusätzlich oder alternativ kann das Abschlussstück 9 zwei jeweils im Wesentlichen parallel zu den Schenkel 3.2.1 des Klapprahmens 3.2 verlaufende Schlitze 9.2 aufweisen, die die Biegsamkeit des Abschlussstücks 9 weiter unterstützen können.

Denkbar ist auch, dass das Abschlussstück 9 eine erste bzw. untere Ausnehmung 9.3 aufweist, die ein verbessertes Einsetzen und Entnehmen des Anzeige- und Bedienmoduls 2 in bzw. aus dem Klapprahmen 3.2 ermöglicht.

Auch weisen die handelsüblichen Smartphones eine integrierte Kamera mit einem Objektiv auf, das im Wesentlichen an einer gleichen Stelle nahe dem oberen Rand auf der Rückseite des Smartphones angeordnet ist.

Um die Funktion der Kamera auch in einem in der Halteeinheit 3 integrierten Zustand des Smartphones nutzen zu können, ragt das integrierte Smartphone im zugeklappten Zustand der Halteeinheit 3 über den Basisrahmen 3.1 hinaus, so dass ein an einen oberen Rand des Smartphones angrenzender Bereich, in dem das Objektiv einer integrierten Kamera angeordnet ist, durch den Basisrahmen 3.1 nicht abgedeckt wird. Somit wird es dem Kontrollbediensteten ermöglicht, vor oder nach erfolgter Fingerabdruckaufnahme ein dazugehöriges Foto von einem Verdächtigen bzw. Einreisenden zu machen.

Im Falle des Vorsehens des Abschlussstücks 9 kann zusätzlich oder alternativ zur ersten bzw. unteren Ausnehmung 9.3 eine zweite bzw. obere Ausnehmung 9.4 aufweist, so dass der an dem oberen Rand des Smartphones angrenzende Bereich, in dem das Objektiv der integrierten Kamera angeordnet ist, durch das Abschlussstücks 9 nicht abgedeckt wird.

Denkbar ist auch, dass das Bildaufnahmemodul 1 über eine in dem Anzeige- und Bedienmodul 2 vorhandene Stromversorgung gespeist wird. Im Basisrahmen 3.1 und/oder im Bildaufnahmemodul 1 kann ein elektronischer Magnetsensor auf einer USB-Leiterplatte (nicht dargestellt) angeordnet sein. Im Klapprahmen 3.2 ist ein Stiftmagnet in einer entsprechenden Bezugsposition zum Magnetsensor angeordnet. Hierdurch wird bewirkt, dass beim Zuklappen des Klapprahmens 3.2, wenn das Bildaufnahmemodul 1 nicht verwendet wird, der entsprechende Magnetsensor auf dem Basisrahmen 3.1 und/oder dem Bildaufnahmemodul 1 über einen elektronischen Schalter, gebildet durch den Magnetsensor und den Stiftmagneten, automatisch die Stromzufuhr des Bildaufnahmemoduls 1 unterbricht, wodurch eine energieeffizientere Vorrichtung realisiert wird.

Denkbar ist, dass der Basisrahmen 3.1, insbesondere an dessen Außenseiten, an der die Vertiefungen ausgebildet sind, eine Schnittstelle aufweist, die es ermöglicht, das in dem Klapprahmen angeordnete Anzeige- und Bedienmodul 2 mit Energie, insbesondere elektrischer Energie, zu versorgen. Zusätzlich oder alternativ ist denkbar, dass über eine zu der Schnittstelle verbundene externe Rechenvorrichtung, insbesondere einen Computer, Daten von der externen Rechenvorrichtung zu dem Anzeige- und Bedienmodul 2 zu senden und/oder von diesem zu empfangen.

### Bezugszeichenliste

1 Bildaufnahmemodul
1.1 Auflagefläche
2 Anzeige- und Bedienmodul
2.1 Display
3 Halteeinheit
3.1 Basisrahmen
3.2 Klapprahmen
3.2.1 Schenkel
4 Drehachse/Zapfen
5 Rastelement
7 Adapterhülle
8 Rastscheibe
9 Abschlussstück
9.1 Schraubverbindungen
9.2 Schlitze
9.3 erste bzw. untere Ausnehmung
9.4 zweite bzw. obere Ausnehmung

## Patentansprüche

1. Handhaltbare Vorrichtung zur Aufnahme von lebenden Hautbereichen menschlicher Autopodien und von Dokumenten,
- wobei die Vorrichtung ein Bildaufnahmemodul (1), das eine Auflagefläche (1.1) aufweist, und eine Halteeinheit (3) für ein Anzeige- und Bedienmodul (2) aufweist, wobei das Anzeige- und Bedienmodul (2) ein Display (2.1) aufweist,
**dadurch gekennzeichnet, dass**
- die Halteeinheit (3) einen Basisrahmen (3.1) und einen Klapprahmen (3.2) enthält, die über ein mechanisches Drehgelenk miteinander verbunden zueinander klappbar sind,
- wobei in dem Basisrahmen (3.1) das Bildaufnahmemodul (1), mit der Auflagefläche (1.1) dem Klapprahmen (3.2) zugewandt, angeordnet ist und in dem Klapprahmen (3.2) das Anzeige- und Bedienmodul (2), mit dem Display (2.1) dem Basisrahmen (3.1) abgewandt, anordenbar ist, und
- wobei in einem aufgeklappten Zustand der Halteeinheit (3) der Basisrahmen (3.1) und der Klapprahmen (3.2) einen Winkel von kleiner 90° und größer 20° miteinander einschließen und in einem zugeklappten Zustand der Halteeinheit (3) das Bildaufnahmemodul (1) von dem Anzeige- und Bedienmodul (2) abdeckbar ist.

2. Handhaltbare Vorrichtung zur Aufnahme von lebenden Hautbereichen menschlicher Autopodien und von Dokumenten nach Anspruch 1, **dadurch gekennzeichnet, dass** das mechanische Drehgelenk durch eine Drehachse (4) und/oder zwei Zapfen (4) gebildet ist, die mit dem Klapprahmen (3.2) fest verbunden sind und in dem Basisrahmen (3.1), insbesondere in dort eingesetzten Rastscheiben (8), gelagert sind.

3. Handhaltbare Vorrichtung zur Aufnahme von lebenden Hautbereichen menschlicher Autopodien und von Dokumenten nach Anspruch 2, **dadurch gekennzeichnet, dass** wenigstens ein Rastelement (5) mit einer Wirkungsrichtung parallel zur Drehachse (4) vorhanden ist, das entweder im Klapprahmen (3.2) gelagert und in den Basisrahmen (3.1) eingreifend oder im Basisrahmen (3.1) gelagert und in den Klapprahmen (3.2) eingreifend angeordnet ist.

4. Handhaltbare Vorrichtung zur Aufnahme von lebenden Hautbereichen menschlicher Autopodien und von Dokumenten nach Anspruch 3, **dadurch gekennzeichnet, dass** das wenigstens eine Rastelement (5) ein federndes Druckstück mit Kugel ist.

5. Handhaltbare Vorrichtung zur Aufnahme von lebenden Hautbereichen menschlicher Autopodien und von Dokumenten nach einem der vorangehenden Ansprüche, **dadurch gekennzeichnet, dass** im zugeklappten Zustand der Halteeinheit (3) der Klapprahmen (3.2) zumindest teilweise innerhalb des Basisrahmens (3.1) liegt.

6. Handhaltbare Vorrichtung zur Aufnahme von lebenden Hautbereichen menschlicher Autopodien und von Dokumenten nach einem der vorangehenden Ansprüche, **dadurch gekennzeichnet, dass** der Klapprahmen (3.2) U-förmig mit zwei zueinander parallelen Schenkeln (3.2.1) ausgeführt ist, zwischen denen das Anzeige- und Bedienmodul (2) anordenbar.

7. Handhaltbare Vorrichtung zur Aufnahme von lebenden Hautbereichen menschlicher Autopodien und von Dokumenten nach Anspruch 6, **dadurch gekennzeichnet, dass** der Klapprahmen (3.1) an seiner Oberkante ein zum Klapprahmen 3.1 verbundenes Abschlussstück (9) aufweist, das mit den zwei zueinander parallelen Schenkeln (3.2.1) des Klapprahmens einen O-förmigen Querschnitt bildet.

8. Handhaltbare Vorrichtung zur Aufnahme von lebenden Hautbereichen menschlicher Autopodien und von Dokumenten nach Anspruch 7, **dadurch gekennzeichnet, dass** das Abschlussstück (9)
- so biegsam ausgeführt ist, dass es zum Einsetzen und Entnehmen des Anzeige- und Bedienmoduls (2) in bzw. aus dem Klapprahmen (3.2) aus einer Einsetz- bzw. Entnahmerichtung des Anzeige- und Bedienmoduls (2) gebogen werden kann,
- zwei jeweils im Wesentlichen parallel zu den Schenkeln (3.2.1) des Klapprahmens (3.2) verlaufende Schlitze (9.2) aufweist,
- eine erste Ausnehmung (9.3) aufweist, die zumindest teilweise in einer unteren Hälfte des Abschlussstücks (9) angeordnet ist, und/oder
- eine zweite Ausnehmung (9.4) aufweist, die zumindest teilweise in einer oberen Hälfte des Abschlussstücks (9) angeordnet ist.

9. Handhaltbare Vorrichtung zur Aufnahme von lebenden Hautbereichen menschlicher Autopodien und von Dokumenten nach einem der vorangehenden Ansprüche, **dadurch gekennzeichnet, dass** das Anzeige- und Bedienmodul 2 ein Smartphone ist.

10. Handhaltbare Vorrichtung zur Aufnahme von lebenden Hautbereichen menschlicher Autopodien und von Dokumenten nach Anspruch 9, **dadurch gekennzeichnet, dass** eine Adapterhülle (7) vorhanden ist, in der das Smartphone eingesetzt, mittelbar im Klapprahmen (3.2) anordenbar ist.

11. Handhaltbare Vorrichtung zur Aufnahme von lebenden Hautbereichen menschlicher Autopodien und von Dokumenten nach Anspruch 10, **dadurch gekennzeichnet, dass** die Adapterhülle (7) aus einem biegsamen Material gefertigt ist und mit dem Klapprahmen (3.2) kraftschlüssig verbunden ist.

12. Handhaltbare Vorrichtung zur Aufnahme von lebenden Hautbereichen menschlicher Autopodien und von Dokumenten nach einem der Ansprüche 9 bis 11, **dadurch gekennzeichnet, dass** das Smartphone so anordenbar ist, dass es im zugeklappten Zustand der Halteeinheit (3) über den Basisrahmen (3.1) hinausragt, so dass ein an einen oberen Rand auf der Rückseite des Anzeige- und Bedienmoduls (2) angrenzender Bereich, in dem ein Objektiv einer integrierten Kamera des Smartphones angeordnet ist, durch den Basisrahmen (3.1) nicht abgedeckt wird.

13. Handhaltbare Vorrichtung zur Aufnahme von lebenden Hautbereichen menschlicher Autopodien und von Dokumenten nach einem der vorangehenden Ansprüche, **dadurch gekennzeichnet, dass** an dem Basisrahmen (3.1) Vertiefungen ausgebildet sind, die so angeordnet und ausgebildet sind, dass ein die Vorrichtung Haltender mit seinen Fingern in diese greifen kann, und/oder an einer Unterseite des Basisrahmens 3.1 eine Schnittstelle für eine Haltevorrichtung, insbesondere für einen Kameragriff, vorgesehen ist, die es dem Haltenden der Vorrichtung ermöglicht, die Vorrichtung zu halten.

14. Handhaltbare Vorrichtung zur Aufnahme von lebenden Hautbereichen menschlicher Autopodien und von Dokumenten nach einem der vorangehenden Ansprüche, **dadurch gekennzeichnet, dass** im Basisrahmen (3.1) und/oder im Bildaufnahmemodul (1) ein elektronischer Magnetsensor und im Klapprahmen (3.2) ein dem Magnetsensor zugeordneter Stiftmagnet vorhanden sind, womit in dem zugeklappten Zustand der Halteeinheit (3) das Bildaufnahmemodul (1) von einer im Anzeige- und Bedienmodul (2) vorhandenen Stromversorgung getrennt wird.

15. System zur Aufnahme von lebenden Hautbereichen menschlicher Autopodien und von Dokumenten,
- wobei das System eine handhaltbare Vorrichtung zur Aufnahme von lebenden Hautbereichen menschlicher Autopodien und von Dokumenten, insbesondere nach einem der vorangehenden Ansprüche, und ein Anzeige- und Bedienmodul (2), das ein Display (2.1) aufweist, aufweist,
- wobei die Vorrichtung ein Bildaufnahmemodul (1), das eine Auflagefläche (1.1) aufweist, und eine Halteeinheit (3) aufweist, **dadurch gekennzeichnet, dass**
- die Halteeinheit (3) einen Basisrahmen (3.1) und einen Klapprahmen (3.2) enthält, die über ein mechanisches Drehgelenk miteinander verbunden zueinander klappbar sind,
- wobei in dem Basisrahmen (3.1) das Bildaufnahmemodul (1), mit der Auflagefläche (1.1) dem Klapprahmen (3.2) zugewandt, angeordnet ist und in dem Klapprahmen (3.2) das Anzeige- und Bedienmodul (2), mit dem Display (2.1) dem Basisrahmen (3.1) abgewandt, angeordnet ist, und
- wobei in einem aufgeklappten Zustand der Halteeinheit (3) die Auflagefläche (1.1) und das Display (2.1) einen Winkel von kleiner 90° und größer 20° miteinander einschließen und in einem zugeklappten Zustand der Halteeinheit (3) das Bildaufnahmemodul (1) von dem Anzeige- und Bedienmodul (2) abgedeckt ist.

## Claims

1. A handheld device for taking images of living skin regions of human autopodia and of documents,
- wherein the device comprises an image recording module (1) having a contact surface (1.1) and a holding unit (3) for a display and control module (2), wherein the display and control module (2) comprises a display (2.1), **characterized in that**
- the holding unit (3) includes a base frame (3.1) and a hinged frame (3.2), which are connected to each other via a mechanical swivel joint and can be folded toward each other,
- wherein the image recording module (1) is arranged in the base frame (3.1), with its contact surface (1.1) facing the hinged frame (3.2), and the display and control module (2) can be arranged within the hinged frame (3.2) with its display (2.1) facing away from the base frame (3.1), and
- wherein, when the holding unit (3) is in an open state, the base frame (3.1) and the hinged frame (3.2) form an angle with each other that is less than 90° and greater than 20°, and when the holding unit (3) is in the closed state, the image recording module (1) can be covered by the display and control module (2).

2. The handheld device for taking images of living skin regions of human autopodia and of documents according to claim 1, **characterized in that** the mechanical swivel joint is formed by a pivot (4) and/or two pins (4) that are fixedly connected to the hinged frame (3.2) and are mounted in the base frame (3.1), in particular in locking discs (8) inserted therein.

3. The handheld device for taking images of living skin regions of human autopodia and of documents according to claim 2, **characterized in that** at least one locking element (5) is provided, with a direction of action parallel to the pivot (4), which locking element (5) is either mounted in the hinged frame (3.2) and arranged to engage with the base frame (3.1), or is mounted in the base frame (3.1) and arranged to engage with the hinged frame (3.2).

4. The handheld device for taking images of living skin regions of human autopodia and of documents according to claim 3, **characterized in that** the at least one locking element (5) is a spring-loaded pressure piece with a ball.

5. The handheld device for taking images of living skin regions of human autopodia and of documents according to any one of the preceding claims, **characterized in that**, when the holding unit (3) is closed, the hinged frame (3.2) lies at least partially within the base frame (3.1).

6. The handheld device for taking images of living skin regions of human autopodia and of documents according to any one of the preceding claims, **characterized in that** the hinged frame (3.2) is U-shaped with two parallel legs (3.2.1), between which the display and control module (2) can be arranged.

7. The handheld device for taking images of living skin regions of human autopodia and of documents according to claim 6, **characterized in that** the hinged frame (3.1) has, at its upper edge, an end piece (9) connected to the hinged frame 3.1, which, together with the two parallel legs (3.2.1) of the hinged frame, forms an O-shaped cross-section.

8. The handheld device for taking images of living skin regions of human autopodia and of documents according to claim 7, **characterized in that** the end piece (9)
- is configured to be flexible enough that it can be bent in the direction of insertion or removal of the display and control module (2) to allow the display and control module (2) to be inserted into or removed from the hinged frame (3.2),
- has two slots (9.2), each of which extends substantially parallel to the legs (3.2.1) of the hinged frame (3.2),
- has a first recess (9.3) that is arranged at least partially in a lower half of the end piece (9), and/or
- has a second recess (9.4) that is arranged at least partially in an upper half of the end piece (9).

9. The handheld device for taking images of living skin regions of human autopodia and of documents according to any one of the preceding claims, **characterized in that** the display and control module 2 is a smartphone.

10. The handheld device for taking images of living skin regions of human autopodia and of documents according to claim 9, **characterized in that** an adapter sleeve (7) is provided, into which the smartphone can be inserted and indirectly positioned within the hinged frame (3.2).

11. The handheld device for taking images of living skin regions of human autopodia and of documents according to claim 10, **characterized in that** the adapter sleeve (7) is made of a flexible material and is connected to the hinged frame (3.2) by a force-fit connection.

12. The handheld device for taking images of living skin regions of human autopodia and of documents according to any one of claims 9 to 11, **characterized in that** the smartphone can be positioned such that, when the holding unit (3) is closed, the smartphone protrudes from the base frame (3.1) so that the base frame (3.1) does not cover an area adjacent to an upper edge on the back of the display and control module (2) in which a lens of an integrated camera of the smartphone is located.

13. The handheld device for taking images of living skin regions of human autopodia and of documents according to any one of the preceding claims, **characterized in that** recesses are formed on the base frame (3.1), which recesses are arranged and configured such that a user holding the device can grasp them with their fingers, and/or an interface for a holding device, in particular for a camera grip, is provided on a bottom surface of the base frame (3.1), which enables the user holding the device to hold the device.

14. The handheld device for taking images of living skin regions of human autopodia and of documents according to any one of the preceding claims, **characterized in that** an electronic magnetic sensor is present in the base frame (3.1) and/or in the image recording module (1), and a bar magnet associated with the magnetic sensor is present in the hinged frame (3.2), whereby, when the holding unit (3) is closed, the image recording module (1) is disconnected from a power supply present in the display and control module (2).

15. A system for taking images of living skin regions of human autopodia and of documents,
- wherein the system comprises a handheld device for taking images of living skin regions of human autopodia and of documents, in particular according to any one of the preceding claims, and a display and control module (2) comprising a display (2.1),
- wherein the device comprises an image recording module (1) having a contact surface (1.1) and a holding unit (3),
**characterized in that**
- the holding unit (3) comprises a base frame (3.1) and a hinged frame (3.2), which are connected to each other via a mechanical swivel joint and can be folded toward each other,
- wherein the image recording module (1) is arranged in the base frame (3.1), with its contact surface (1.1) facing the hinged frame (3.2), and the display and control module (2) is arranged in the hinged frame (3.2) with its display (2.1) facing away from the base frame (3.1), and
- wherein, when the holding unit (3) is in an open state, the contact surface (1.1) and the display (2.1) form an angle with each other that is less than 90° and greater than 20°, and when the holding unit (3) is in a closed state, the image recording module (1) is covered by the display and control module (2).

## Revendications

1. Dispositif portatif de prise d'images de régions cutanées vivantes de documents et d'autopodes humains,
- le dispositif comprenant un module de prise d'images (1) doté d'une surface d'appui (1.1) et une unité de fixation (3) pour un module d'affichage et de commande (2), le module d'affichage et de commande (2) comportant un écran (2.1),
**caractérisé en ce que**
- l'unité de fixation (3) comprend un cadre de base (3.1) et un cadre rabattable (3.2), qui sont reliés entre eux par une articulation mécanique et peuvent être rabattus l'un vers l'autre,
- le module de prise d'images (1) étant disposé dans le cadre de base (3.1) de telle sorte que sa surface d'appui (1.1) soit tournée vers le cadre rabattable (3.2), et le module d'affichage et de commande (2) pouvant être disposé dans le cadre rabattable (3.2) de telle sorte que son écran (2.1) soit détourné du cadre de base (3.1), et
- le cadre de base (3.1) et le cadre rabattable (3.2) formant entre eux un angle inférieur à 90° et supérieur à 20° lorsque l'unité de fixation (3) est déployée, et le module de prise d'images (1) pouvant être recouvert par le module d'affichage et de commande (2) lorsque l'unité de fixation (3) est repliée.

2. Dispositif portatif de prise d'images de régions cutanées vivantes de documents et d'autopodes humains selon la revendication 1, **caractérisé en ce que** l'articulation mécanique est constituée d'un axe de rotation (4) et/ou de deux tourillons (4) qui sont solidaires du cadre rabattable (3.2) et qui sont montés dans le cadre de base (3.1), notamment dans des disques d'encliquetage (8) qui y sont insérés.

3. Dispositif portatif de prise d'images de régions cutanées vivantes de documents et d'autopodes humains selon la revendication 2, **caractérisé en ce qu'**il existe au moins un élément d'encliquetage (5) dont la direction d'action est parallèle à l'axe de rotation (4), lequel est soit monté dans le cadre rabattable (3.2) et s'engage dans le cadre de base (3.1), soit monté dans le cadre de base (3.1) et s'engage dans le cadre rabattable (3.2).

4. Dispositif portatif de prise d'images de régions cutanées vivantes de documents et d'autopodes humains selon la revendication 3, **caractérisé en ce que** ledit au moins un élément d'encliquetage (5) est une pièce de pression élastique munie d'une bille.

5. Dispositif portatif de prise d'images de régions cutanées vivantes de documents et d'autopodes humains selon l'une quelconque des revendications précédentes, **caractérisé en ce que**, lorsque l'unité de fixation (3) est repliée, le cadre rabattable (3.2) se trouve au moins en partie à l'intérieur du cadre de base (3.1).

6. Dispositif portatif de prise d'images de régions cutanées vivantes de documents et d'autopodes humains selon l'une quelconque des revendications précédentes, **caractérisé en ce que** le cadre rabattable (3.2) est réalisé en forme de U avec deux branches parallèles (3.2.1), entre lesquelles le module d'affichage et de commande (2) peut être disposé.

7. Dispositif portatif de prise d'images de régions cutanées vivantes de documents et d'autopodes humains selon la revendication 6, **caractérisé en ce que** le cadre rabattable (3.1) comporte, au niveau de son bord supérieur, un embout (9) relié au cadre rabattable 3.1, qui forme, avec les deux branches parallèles (3.2.1) du cadre rabattable, une section transversale en forme de O.

8. Dispositif portatif de prise d'images de régions cutanées vivantes de documents et d'autopodes humains selon la revendication 7, **caractérisé en ce que** l'embout (9)
- est conçu de manière suffisamment souple pour pouvoir être plié dans le sens d'insertion ou de retrait du module d'affichage et de commande (2) afin de permettre son insertion dans le cadre rabattable (3.2) ou son retrait de celui-ci,
- comporte deux fentes (9.2) s'étendant chacune essentiellement parallèlement aux branches (3.2.1) du cadre rabattable (3.2),
- comporte un premier évidement (9.3) situé au moins en partie dans une moitié inférieure de l'embout (9), et/ou
- comporte un deuxième évidement (9.4) situé au moins en partie dans une moitié supérieure de l'embout (9).

9. Dispositif portatif de prise d'images de régions cutanées vivantes de documents et d'autopodes humains selon l'une quelconque des revendications précédentes, **caractérisé en ce que** le module d'affichage et de commande 2 est un smartphone.

10. Dispositif portatif de prise d'images de régions cutanées vivantes de documents et d'autopodes humains selon la revendication 9, **caractérisé en ce qu'**il existe un manchon adaptateur (7) dans lequel le smartphone peut être inséré et ainsi être positionné indirectement dans le cadre rabattable (3.2).

11. Dispositif portatif de prise d'images de régions cutanées vivantes de documents et d'autopodes humains selon la revendication 10, **caractérisé en ce que** la coque d'adaptation (7) est réalisée dans un matériau souple et est reliée par adhérence au cadre rabattable (3.2).

12. Dispositif portatif de prise d'images de régions cutanées vivantes de documents et d'autopodes humains selon l'une quelconque des revendications 9 à 11, **caractérisé en ce que** le smartphone peut être positionné de telle sorte que, lorsque l'unité de fixation (3) est repliée, le smartphone dépasse du cadre de base (3.1), de sorte qu'une zone adjacente à un bord supérieur à l'arrière du module d'affichage et de commande (2), dans laquelle est disposé un objectif d'une caméra intégrée au smartphone, ne soit pas recouverte par le cadre de base (3.1).

13. Dispositif portatif de prise d'images de régions cutanées vivantes de documents et d'autopodes humains selon l'une quelconque des revendications précédentes, **caractérisé en ce que** le cadre de base (3.1) comporte des renfoncements qui sont disposés et réalisés de manière à ce qu'une personne tenant le dispositif puisse y glisser ses doigts, et/ou qu'une interface destinée à un dispositif de fixation, notamment une poignée de caméra, soit prévue sur une face inférieure du cadre de base 3.1, permettant ainsi à la personne tenant le dispositif de le maintenir.

14. Dispositif portatif de prise d'images de régions cutanées vivantes de documents et d'autopodes humains selon l'une quelconque des revendications précédentes, **caractérisé en ce qu'**il existe un capteur magnétique électronique dans le cadre de base (3.1) et/ou dans le module de prise d'images (1) et qu'il existe, dans le cadre rabattable (3.2) un aimant cylindrique associé au capteur magnétique, ce qui permet, lorsque l'unité de fixation (3) est repliée, de déconnecter le module de réception d'image (1) d'une alimentation électrique présente dans le module d'affichage et de commande (2).

15. Système de prise d'images de régions cutanées vivantes de documents et d'autopodes humains,
- le système comprenant un dispositif portatif de prise d'images de régions cutanées vivantes de documents et d'autopodes humains, notamment selon l'une quelconque des revendications précédentes, ainsi qu'un module d'affichage et de commande (2) comportant un écran (2.1),
- le dispositif comprenant un module de prise d'images (1) doté d'une surface d'appui (1.1) et une unité de fixation (3),
**caractérisé en ce que**
- l'unité de fixation (3) comprend un cadre de base (3.1) et un cadre rabattable (3.2), qui sont reliés entre eux par une articulation mécanique et peuvent être rabattus l'un vers l'autre,
- le module de prise d'images (1) étant disposé dans le cadre de base (3.1) de telle sorte que sa surface d'appui (1.1) soit tournée vers le cadre rabattable (3.2), et le module d'affichage et de commande (2) étant disposé dans le cadre rabattable (3.2) de telle sorte que son écran (2.1) soit détourné du cadre de base (3.1), et
- la surface d'appui (1.1) et l'écran (2.1) formant, lorsque l'unité de fixation (3) est déployée, un angle compris entre moins de 90° et plus de 20°, et le module de prise d'images (1) étant masqué par le module d'affichage et de commande (2) lorsque l'unité de fixation (3) est repliée.
